# EUROPEAN PATENT SPECIFICATION

(11) **EP 4 403 589 B1**
(45) Date of publication and mention of the grant of the patent: **15.04.2026**
(21) Application number: 23152513.0
(22) Date of filing: 19.01.2023
(51) Int. Cl.: C08G 18/76, C08J 11/28

(54) **A PROCESS FOR PREPARING AT LEAST ONE POLYISOCYANATE FROM SOLID MATERIAL W**
VERFAHREN ZUR HERSTELLUNG VON MINDESTENS EINEM POLYISOCYANAT AUS FESTEM MATERIAL W
PROCÉDÉ DE PRÉPARATION D'AU MOINS UN POLYISOCYANATE À PARTIR DE MATÉRIAU SOLIDE W

(43) Date of publication of application: 24.07.2024
(73) Proprietor: BASF SE, 67056 Ludwigshafen am Rhein (DE)
(72) Inventor: VANDEWALLE, Koenraad, 2040 Antwerpen (BE); FRIEDRICH, Holger, 67056 Ludwigshafen am Rhein (DE); FERBITZ, Jens, 67056 Ludwigshafen am Rhein (DE); MATTKE, Torsten, 67056 Ludwigshafen am Rhein (DE)
(74) Representative: BASF IP Association

(56) References cited:
- EP-A1- 0 976 719
- WO-A1-2012/077199
- KR-A- 20130 120 551

## Description

The present invention relates to a process for preparing at least one polyisocyanate from a solid material W.

Di- and polyisocyanates are used in particular in the production of polyurethanes and coatings. Most widely used are aromatic di- and polyisocyanates such as TDI and MDI. Besides, both aliphatic and cycloaliphatic di- and polyisocyanates are also known. Preparation thereof is usually done by phosgenation of the corresponding amines. Phosgene-free routes, for example via reductive or oxidative carbonylation, are also known, but play only a minor technical role. Preparation of phosgene is usually done from chlorine and CO in a heterogeneously catalyzed gas phase reaction. In the phosgenation of the amines, HCl is formed as a by-product. In addition to its use in other processes (e.g. oxychlorination), processes for the recovery of chlorine from HCl are also known (electrolysis or Deacon process).

In the course of developing sustainable preparation processes, efforts are being made to use renewable raw materials, recycled materials or CO₂ itself. For example, WO2021/089737 discloses a process for the preparation of isocyanates by phosgenation of the corresponding amines, wherein the phosgene is produced from CO and chlorine and the CO is obtained from CO₂ by a reverse water gas shift (RWGS) reaction. The hydrogen required for this is provided by water electrolysis, whereby excess hydrogen can also be used in the production of the amines by hydrogenation of the nitro components. According to the present invention, the oxygen produced in the water electrolysis can be used in an oxidation of HCl (electrochemically or heterogeneous catalytic) to chlorine, which in turn can be used in phosgene synthesis. In a preferred embodiment, the source of CO₂ is the combustion of polyurethane waste or pyrolysis products of polyurethane waste.

WO2020/239716 discloses the pyrolysis of a PU waste material in the course of which synthesis gas (CO/H₂), CO₂, low-molecular hydrocarbons and a residue are obtained. The CO₂ is converted in an electrolysis to CO and oxygen, whereby the oxygen is used for the combustion of the above-mentioned residue and possibly further amounts of PU waste material. The resulting CO₂ is also fed to the above-mentioned CO₂ electrolysis. The resulting CO is used again in the synthesis of phosgene for the production of isocyanates.

EP 0 976 719 A1 discloses an apparatus for hydrolyzing and recycling polyisocyanate derivatives having at least one isocyanato group and/or a group derived from an isocyanato group as target compounds to be hydrolyzed into raw materials or derivatives thereof for the target compounds.

WO 2012/077199 A1 discloses a mixed gas production device and a mixed gas production system that are able to adjust the ratio of the quantities of CO and H₂ produced.

KR 2013 0120551 A discloses a polyisocyanate production method that can allow effective use of hydrogen chloride produced secondarily in a polyisocyanate production process, while allowing reduction of environmental burdens, and a polyisocyanate production system for performing the polyisocyanate production method.

There is however still a need to provide processes with improved sustainability.

Therefore, it was an object of the present invention to provide an improved process for preparing polyisocyanates which exhibits good performance while showing improved sustainability compared to processes already known in the art. Indeed, it was an object of the present invention to provide a process for preparing polyisocyanates with reduced environmental impact while being cost effective.

Therefore, the present invention relates to a process for preparing at least one polyisocyanate from a solid material W, the process comprising
(i) providing the solid material W comprising at least one polymer, selected from the group consisting of polyurethane, polyurethane urea, polyisocyanurate and a mixture thereof;
(ii) preparing at least one polyamine from W, comprising:
   passing and subjecting W provided according to (i) to solvolysis reaction conditions into a solvolysis zone R_{S} comprising one or more reactor units, obtaining a stream S_{PA} comprising the corresponding at least one polyamine, a stream SL comprising at least one polyol and a stream S1 comprising CO₂;
(iii) passing S1 obtained according to (ii) through a CO₂-reduction unit U_{R}, obtaining a stream S_{CO} comprising CO;
(iv) preparing phosgene, comprising
   (iv.1) providing a stream S2 comprising Cl₂;
   (iv.2) passing S_{CO} obtained according to (iii) into a reaction unit for preparing phosgene RU_{P} and contacting S_{CO} with S2 obtained according to (iv.1) into RU_{P}, obtaining a stream S3 comprising phosgene;
(v) passing the at least one polyamine obtained according to (ii) as S_{PA} into a reaction unit RU and reacting S_{PA} with phosgene comprised in S3 obtained according to (iv.2), obtaining a stream SP comprising the corresponding at least one polyisocyanate;
   wherein, in SP, the at least one polyisocyanate is TDI or MDI.

Preferably, the solid material W is a waste solid material. For example, the waste material can be one or more of an end-of-life material, such as an end-of-life foam, end-of-life rigid foam, end-of-life flexible foam, an end-of-life compact elastomer, end-of-life compact duromer and end-of-life semi-rigid foam. In the context of the present invention, an end-of-life material is a material at the end of its lifecycle.

It is possible that the solid material W comprises, in addition to the one or more polymers, impurities which can be one or more of glass, sand, wood, metals, papers, inorganic solids and polymers other than polyurethanes, polyurethane ureas and polyisocyanurates. The polymers other than polyurethane, polyurethane urea and polyisocyanurate can be for example one or more of polyethylene (PE), polypropylene (PP), polyethylene terephthalate (PET) or polystyrene (PS).

Preferably, the solid material W is one or more of a powder, pieces of foam, pellets, granulates and flakes.

Preferably, the average particle size of the solid material W provided in (i) is in the range of from 10⁻⁶ to 10⁻¹ m, preferably in the range of from 10⁻⁶ to 10⁻² m, the average particle size being determined by laser diffraction or light microscopy. These methods being adapted by the skilled person depending on the particle size range. Thus, particle sieve analysis could also be used for determining the average particle size.

Optionally, the process further comprises, prior to (i), subjecting the solid material W to a pre-treatment, preferably a mechanical pre-treatment, wherein more preferably the mechanical pre-treatment comprises one or more of milling, crushing, shredding and cutting, the solid material W.

Preferably, the process further comprises, prior to (i), subjecting the solid material W to one or more of a washing step and an extraction step.

Prior to (i), or after the pre-treatment and prior to (i), the process may further comprise a purification step such assorting, windsifting, magnetic separation, and/or flotation. These techniques known in the art permit to sort and/or remove impurities in W which are not polyurethane, polyurethane urea and/or polyisocyanurate that could be present in W, such as glass, sand, wood, papers, metals, polymers other than polyurethanes, polyurethane ureas and polyisocyanurates.

### Step (ii) - Solvolysis

Preferably, (ii) comprises
(ii.1) passing and subjecting W provided according to (i) to hydrolysis reaction conditions into R_{S} being a hydrolysis reaction unit, obtaining a stream S_{PA} comprising the corresponding at least one polyamine, a stream SL comprising at least one polyol and a stream S1 comprising CO₂.

Preferably, the hydrolysis according to (ii.1) is performed at a temperature in the range of from 100 to 340 °C, more preferably in the range of from 150 to 250 °C.

Preferably, the hydrolysis according to (ii.1) is performed at a pressure in the range of from 1 to 150 bar(abs), more preferably in the range of from 1 to 40 bar(abs) .

Preferably, subjecting W provided according to (i) to hydrolysis reaction conditions into R_{S} comprises contacting W with water in the presence of a catalyst, preferably a base. Preferably, the catalyst, preferably the base, is selected from the group consisting of an alkali metal compounds, an alkaline earth metal compound, ammonia, organic amines and a mixture thereof, more preferably an alkali metal compound, more preferably alkali metal hydroxide. More preferably, the catalyst is potassium hydroxide or sodium hydroxide, more preferably potassium hydroxide.

In the context of the present invention, it is noted that any hydrolysis reaction known in the art can be used for performing step (ii) of the process of the present invention. For example, hydrolysis can be performed as described in Gregory A. Campbell et.al. "Polyurethane Foam recycling - Superheated steam hydrolysis", Environmental Science & Technology, Vol.10 (2), 1976, US 2003/0012710 A, US 5208379 A, US 4317939 A.

As an alternative, preferably, (ii) comprises
(ii.1a)passing and subjecting W provided according to (i) to hydroaminolysis reaction conditions into R_{S} being a hydroaminolysis reaction unit, obtaining a stream S_{PA} comprising the corresponding at least one polyamine, a stream SL comprising at least one polyol and a stream S1 comprising CO₂.

Preferably, the hydroaminolysis according to (ii.1a) is performed at a temperature in the range of from 100 to 300 °C, more preferably in the range of from 100 to 250 °C.

Preferably, the hydroaminolysis according to (ii.1a) is performed at a pressure in the range of from 1 to 150 bar(abs), more preferably in the range of from 1 to 40 bar(abs) .

Preferably, subjecting W provided according to (i) to hydroaminolysis reaction conditions into R_{S} comprises contacting W with water in the presence of an amine compound. Preferably, the amine compound is one or more of ammonia, pyridine, imidazole, TDA and MDA.

In the context of the present invention, any hydroaminolysis reaction known in the art can be used for performing step (ii) of the process of the present invention. For example, hydroaminolysis can be performed as described in CN 110105621 A and DE 4217524 A1.

As a further alternative, the solvolysis according to (ii) preferably comprises an aminolysis followed by a hydrolysis.

According to said alternative, preferably, prior to (i), more preferably after subjecting the solid material W to a pre-treatment as defined herein above, drying the solid material W, wherein drying is more preferably conducted at a temperature in the range of from 40 to 100 °C, more preferably in the range of from 50 to 85 °C, and wherein more preferably drying is conducted in a gas atmosphere comprising one or more of nitrogen and oxygen, more preferably air.

Preferably the water content of the solid material W, more preferably after drying as defined herein above, is lower than 1000 ppm-weight-%, more preferably lower than 100 ppm-weight-%, more preferably from 0 to 100 ppm-weight-%.

Preferably, (ii) comprises
(ii.1b.1) passing W provided in (i) and one or more amines in a reactor unit R_{A} comprised in R_{S} and subjecting W and the one or more amines to aminolysis reaction conditions in R_{A}, obtaining a mixture M comprising at least one polyurea-containing compound, and further comprising at least one polyol;
(ii.1b.2) isolating the at least one polyurea-containing compound of M from the at least one polyol obtained according to (ii.1b.1), obtaining M_{PU} comprising the at least one poly-urea-containing compound;
(ii.1b.3) passing water as stream S0 and M_{PU} obtained according to (ii.1b'.2) through an hydrolysis reactor unit R_{H} comprised in R_{S} and located downstream of R_{A}, obtaining a stream S_{PA} comprising the corresponding at least one polyamine and a stream S1 comprising CO₂.

Preferably, the reactor unit R_{A} according to (ii.1b.1) comprises, more preferably consists of, one or more reactors. More preferably, each of the one or more reactors is a stirred reactor, more preferably a stirred tank reactor.

Preferably, the one or more amines used in (ii.1b.1) are selected from the group consisting of one or more primary amines and one or more secondary amines, such as dibutylamine, more preferably the one or more amines used in (ii.1b.1) are one or more primary amines.

Preferably, (ii) comprises
(ii.1b.1) passing W provided in (i) and one or more primary amines in R_{A} comprised in R_{S} and subjecting W and the one or more primary amines to aminolysis reaction conditions in R_{A}, obtaining a mixture M comprising at least one polyurea-containing compound, and further comprising at least one polyol;
(ii.1b.2) isolating the at least one polyurea-containing compound of M from the at least one polyol obtained according to (ii.1b.1), obtaining M_{PU} comprising the at least one poly-urea-containing compound;
(ii.1b.3) passing water as stream S0 and M_{PU} obtained according to (ii.1b.2) through R_{H} comprised in R_{S} and located downstream of R_{A}, obtaining a stream S_{PA} comprising the corresponding at least one polyamine and a stream S1 comprising CO₂.

Preferably, the one or more primary amines used in (ii.1b.1) are free of hydroxyl groups.

Preferably the one or more primary amines used in (ii.1b.1) are free of hydroxyl groups and free of aldehyde groups. More preferably the one or more primary amines used in (ii.1b.1) are free of hydroxyl groups, free of aldehyde groups and free of ketone groups.

In other words, preferably the atoms forming the one or more primary amines used in (ii.1b.1) are C, H and N.

Preferably, the one or more primary amines used in (ii.1b.1) are selected from the group consisting of aliphatic monoamines, aliphatic polyamines, aromatic monoamines, aromatic polyamines, and mixtures of two or more thereof, more preferably from the group consisting of aliphatic monoamines, aromatic monoamines, and mixtures of two thereof.

Preferably, the one or more primary amines used in (ii.1b.1) are aliphatic monoamines, preferably the aliphatic monoamines are selected from the group consisting of n-butylamines, cyclohexylamines, n-octylamines, n-hexylamines, n-propylamines, n-dodecylamines, n-tridecyla-mines, n-octadecylamines, and mixtures of two or more thereof, more preferably selected from the group consisting of n-butylamines, n-octylamines, n-hexylamines and n-propylamines, more preferably the aliphatic monoamines are n-butylamines.

Alternatively, preferably, the one or more primary amines used in (ii.1b.1) are aromatic monoamines, wherein the aromatic monoamines are selected from the group consisting of aniline, toluidine, naphtylamine, and mixtures of two or more thereof, wherein the aromatic monoamines preferably are aniline.

Alternatively, preferably, the one or more primary amines used in (ii.1b.1) are aliphatic polyamines, wherein the aliphatic polyamines are selected from the group consisting of hexamethylendiamine, ethylenediamine, propanediamine, such as propane-1,3-diamine, propane-1,2-diamine, isophorone diamine, butanediamine, such as butane-1,4-diamine, butane-1,3-diamine, pentadiamine, pentane-1,5-diamine, diaminocyclohexane, such as 1,2-diaminocyclohexane, and a mixture of two or more thereof, more preferably selected from the group consisting of hexamethylendiamine, ethylenediamine, propanediamine and butanediamine, more preferably selected from the group consisting of hexamethylendiamine, ethylenediamine, and propanediamine; wherein the aliphatic polyamines more preferably are selected from the group consisting of ethylendiamine and propanediamines.

In the context of the present invention, all isomers of the aforementioned aliphatic polyamines can be envisaged. Preferably, propanediamine is propane-1,3-diamine or propane-1,2-diamine.

Alternatively, preferably, the one or more primary amines used in (ii.1b.1) are aromatic polyamines, more preferably the aromatic polyamines are selected from the group consisting of diaminotoluene, phenylenediamine and diaminodiphenylmethane, more preferably selected from the group consisting of diaminotoluene, phenylenediamine and 4,4'-diaminodiphenylmethane, more preferably is diaminotoluene, more preferably 2,4-diaminotoluene.

More preferably, the one or more primary amines used in (ii.1b.1) are aliphatic monoamines. More preferably, the primary amine used in (ii.1b.1) is n-butylamine.

Preferably, in (ii.1b.1), the ratio of the weight of the solid material W introduced into R_{A} relative to the weight of the one or more amines introduced into R_{A} is in the range of from 1:100 to 1:1, more preferably in the range of from 1:40 to 1:3, more preferably in the range of from 1:27 to 1:5.

Preferably, the aminolysis reaction according to (ii.1b.1) is performed at a temperature in the range of from 50 to 250 °C, more preferably in the range of from 80 to 200 °C, more preferably in the range of from 100 to 220 °C, more preferably in the range of from 120 to 180 °C.

Preferably, the aminolysis reaction according to (ii.1b.1) is performed at a pressure in the range of from 1.0 to 25 bar(abs), more preferably in the range of from 1 to 20 bar(abs), more preferably in the range of from 1 to 18 bar(abs).

Preferably, the aminolysis reaction according to (ii.1b.1) is conducted for a duration in the range of from 1 to 600 min, more preferably in the range of from 20 to 450 min, more preferably in the range of from 40 to 150 min.

Preferably at most 0.1 weight-%, more preferably from 0 to 0.01 weight-%, more preferably from 0 to 0.001 weight-%, of the mixture M obtained according to (ii.1 b.1) consist of polymer selected from the group consisting of polyurethane, polyurethane urea and polyisocyanurate.

In other words, it is preferred that the mixture M is substantially free of, more preferably free of, polymer selected from the group consisting of polyurethane and polyisocyanurate, meaning essentially free of, preferably free of polyurethane and of polyisocyanurate. This is identified by the analysis of the mixture M by IR spectroscopy: The mixture M presents no NCO-stretching vibration at 2200-2400 cm⁻¹ meaning that the reaction (aminolysis) is complete.

Preferably, the process further comprises, after (ii.1b.1) and prior to (ii.1b.2),
removing the mixture M obtained according to (ii.1b.1) from R_{A} and passing M into a solid-liquid separation unit, obtaining a liquid mixture M_{SLS} comprising the one or more polyurea-containing compounds and the one or more polyols and obtaining a solid mixture comprising impurities.

Preferably the impurities are one or more of glass, sand, wood, metals, papers, inorganic solids and polymers other than polyurethanes, polyurethane ureas, polyisocyanurates. The polymers other than polyurethane, polyurethane urea, polyisocyanurate can be for example one or more of polyethylene (PE), polypropylene (PP), polyethylene terephthalate (PET) or polystyrene (PS).

Preferably, the solid-liquid separation unit is a filtration unit or a centrifuge, more preferably a filtration unit, more preferably a filter, more preferably a pocket filter, a bag filter, a membrane filter, a candle filter, an agitated pressure filter, a vacuum belt filter, a frame & plate filter, or a nutsche filter.

Preferably passing the mixture M removed from R_{A} into a solid-liquid separation unit is performed at a temperature in the range of from 50 to 250 °C, more preferably in the range of from 80 to 200 °C, more preferably in the range of from 100 to 220 °C, more preferably in the range of from 140 to 200 °C.

Preferably, the solid-liquid separation is performed at a pressure in the range of from 1.0 to 20.0 bar(abs), more preferably in the range of from 1.0 to 18.0 bar(abs).

Preferably, (ii.1b.2) comprises
(ii.1b.2.1) more preferably passing a solvent and the mixture M, more preferably the liquid mixture M_{SLS} obtained as detailed in the foregoing, into an evaporation unit EU comprised in R_{S}, obtaining from EU a vapor mixture V comprising the solvent and at least a portion of the one or more primary amines, and a liquid mixture M_{EU} comprising the one or more polyurea-containing compounds and the one or more polyols;
(ii.1b.2.2) adding a solvent to the mixture M, more preferably the liquid mixture M_{SLS} obtained as detailed in the foregoing, more preferably the liquid mixture M_{EU} obtained according to (ii.1b.2.1), allowing the one or more polyurea-containing compounds to precipitate, obtaining a mixture comprising one or more precipitated polyurea-containing compounds;
(ii.1b.2.3) passing the mixture comprising the one or more precipitated polyurea-containing compounds obtained according to (ii.1b.2.2) into a solid-liquid separation unit SLU comprised in R_{S}, more preferably SLU being a filter, obtaining a solid mixture M_{PU} comprising the at least one polyurea-containing compound.

Preferably, according to (ii.1b.2.3), in addition to M_{PU}, a liquid mixture M_{LP} comprising at least one polyol is obtained. Preferably, the process further comprises passing M_{LP} through one or more purification unit.

Preferably, the evaporation in EU according to (ii.1b.2.1), is performed at a pressure difference, being the difference between the pressure before the evaporation unit and the pressure at the evaporation unit, being in the range from 0 to 30 bar(abs), preferably in the range of from 0.1 to 25 bar(abs), more preferably in the range of from 0.2 to 20 bar(abs).

Preferably the solvent used in one or more of (ii.1b.2.1) and (ii.1b.2.2), more preferably in (ii.1 b.2.1) and (ii.1b.2.2), has a water content in the range of from 0 to 1000 ppm, more preferably in the range of from 0 to 500 ppm, more preferably in the range of from 0 to 100 ppm. In the context of the present invention, said solvent is thus preferably an anhydrous solvent.

Preferably, the solvent used in one or more of (ii.1b.2.1) and (ii.1b.2.2), more preferably in (ii.1b.2.1) and (ii.1b.2.2), is selected from the group consisting of hydrocarbons, ketones and ether, more preferably selected from the group consisting of xylene, toluene, n-heptane, methyl ethyl ketone (MEK), dioxane, benzene, heptan-2-one and a mixture of two or more thereof, more preferably is selected from the group consisting of xylene, toluene, n-heptane, benzene and a mixture of two or more thereof, more preferably is xylene or toluene or benzene.

Preferably, the evaporation unit EU used in (ii.1b.2.1) is one or more of a reactor equipped with a filter, a fractionated distillation column and a flash drum.

Preferably, for (ii.1b.2.1), the amount of the solvent added is calculated on the basis of the weight ratio of said solvent relative to the at least one polymer of W provided according (i) which is in the range of from 0.1:1 to 50:1, more preferably in the range of from 1:1 to 20:1, more preferably in the range of from 1:1 to 15:1, more preferably in the range of from 1:1 to 10:1.

Preferably, for (ii.1 b.2.2), the amount of the solvent added is calculated on the basis of the weight ratio of said solvent relative to the at least one polymer of W provided according (i) which is in the range of from 0.1:1 to 50:1, more preferably in the range of from 1:1 to 20:1, more preferably in the range of from 1:1 to 15:1, more preferably in the range of from 1:1 to 10:1. Preferably, (ii.1b.2.3) further comprises washing the mixture M_{PU} with a solvent on SLU, wherein the solvent is selected from the group consisting of hydrocarbons, ketones and ether, more preferably selected from the group consisting of benzene, xylene, toluene, n-heptane, methyl ethyl ketone (MEK), dioxane, heptan-2-one and a mixture of two or more thereof, preferably is selected from the group consisting of xylene, toluene, n-heptane, benzene, and a mixture of two or more thereof, more preferably is benzene, xylene or toluene.

Preferably said solvent has a water content in the range of from 0 to 1000 ppm, more preferably in the range of from 0 to 500 ppm, more preferably in the range of from 0 to 100 ppm. In the context of the present invention, said solvent is thus preferably an anhydrous solvent.

Preferably the solvent used in (ii.1b.2.3) is the same as the one used in (ii.1b.2.1) and (ii.1b.2.2).

Preferably at most 0.1 weight-%, more preferably from 0 to 0.01 weight-%, more preferably from 0 to 0.001 weight-%, of the mixture M_{PU}, after washing as mentioned above, consist of polyol. In other words, it is preferred that the mixture M_{PU} be substantially free of, more preferably free of, polyol. This can be for example identified with ¹³C_NMR or HPLC.

Preferably the reactor unit R_{H} comprises one or more reactors.

Preferably, according to (ii.1b.3) the weight ratio of water relative to the at least one polyurea-containing compound comprised in M_{PU} is in the range of from 1:1 to 50:1, more preferably in the range of from 5:1 to 20:1, more preferably in the range of from 10:1 to 18:1.

Preferably, the hydrolysis is performed in R_{S}, more preferably in R_{H} comprised in R_{S}, at a temperature in the range of from 150 to 350 °C, more preferably in the range of from 210 to 290 °C, more preferably in the range of from 230 to 270 °C.

Preferably, the hydrolysis is performed in R_{H} under autogenous pressure, more preferably at a pressure in the range of from 20 to 70 bar(abs), more preferably in the range of from 25 to 65 bar(abs), more preferably in the range of from 30 to 60 bar(abs).

Preferably, (ii.1b.3) further comprises depressurizing of the reaction mixture in R_{H} and one or more distillation steps, obtaining, in addition to stream S_{PA} and S1, a stream S10 comprising water and a stream S5 comprising the one or more primary amines.

Preferably, the process further comprises recycling at least a portion of S10 as stream S0 in (ii.1b.3).

Preferably, the process further comprises recycling at least a portion of S5 for using as the one or more primary amines in (ii.1b.1).

Preferably, (ii.1b.3) further comprises
passing S_{PA} comprising the at least one polyamine through a purification unit, more preferably a distillation column, obtaining a purified stream S_{PA} comprising the at least one polyamine. Alternatively, in the context of the present invention, any aminolysis reaction combined with hydrolysis known in the art can be used for performing step (ii) of the process of the present invention. For example, hydrolysis combined with aminolysis can be performed as described in FR 1429011 A, DE 4217524 A1, EP 1149862 A1 and CN 110105621 A.

As a further alternative, preferably, the solvolysis according to (ii) is a hydroalcoholysis reaction.

According to said alternative, preferably (ii) comprises
(ii.1c) passing and subjecting W provided according to (i) to hydroalcoholysis reaction conditions into R_{S} being a hydroalcoholysis reaction unit, obtaining a stream S_{PA} comprising the corresponding at least one polyamine, a stream SL comprising at least one polyol and a stream S1 comprising CO₂.

Preferably, the hydroalcoholysis is a hydroglycolysis.

Preferably, the hydroalcoholysis according to (ii.1) is performed at a temperature in the range of from 100 to 250 °C, more preferably in the range of from 150 to 220 °C.

Preferably, the hydroalcoholysis according to (ii.1) is performed at a pressure in the range of from 1 to 60 bar(abs), more preferably in the range of from 1 to 20 bar(abs).

Preferably, subjecting W provided according to (i) to hydroalcoholysis reaction conditions into R_{S} comprises contacting W with water in the presence of an alcohol and a catalyst compound. Preferably, the alcohol is one or more of ethylene glycol, diethylene glycol, methanol and ethanol. More preferably, subjecting W provided according to (i) to hydroglycolysis reaction conditions into R_{S} comprises contacting W with water, diethylene glycol in presence of a catalyst, the catalyst being selected from the group consisting of an alkali metal compounds, an alkaline earth metal compound, amines, such as TDA and MDA, and a mixture thereof, more preferably an alkali metal compound, more preferably alkali metal hydroxide. More preferably, the catalyst is potassium hydroxide or sodium hydroxide, more preferably potassium hydroxide.

As a further alternative, preferably, the solvolysis according to (ii) comprises an alcoholysis reaction, more preferably glycolysis reaction, and an hydrolysis reaction.

According to said alternative, preferably (ii) comprises
(ii.1d.1) passing W provided in (i) and one or more alcohols, more preferably diethylene glycol, in a reactor unit R_{G} comprised in R_{S} and subjecting W and the one or more alcohols to alcoholysis reaction conditions, more preferably glycolysis reaction conditions, in R_{A}, obtaining a mixture M comprising at least one carbamate-containing compound, and further comprising at least one polyol;
(ii.1d.2) isolating the at least one carbamate-containing compound of M from the at least one polyol obtained according to (ii.1d.1), obtaining M_{PU} comprising the at least one carbamate-containing compound and a stream SL comprising the at least one polyol;
(ii.1d.3) passing water as stream S0 and M_{PU} obtained according to (ii.1d.2) through an hydrolysis reactor unit R_{H} comprised in R_{S} and located downstream of R_{A}, obtaining a stream S_{PA} comprising the corresponding at least one polyamine and a stream S1 comprising CO₂.

Preferably, the glycolysis according to (ii.1d.1) is performed at a temperature in the range of from 100 to 250 °C, more preferably in the range of from 150 to 220°C.

Preferably, the glycolysis according to (ii.1d.1) is performed at a pressure in the range of from 1 to 60 bar(abs), more preferably in the range of from 1 to 20 bar(abs).

Preferably, in (ii.1d.1), passing W provided according to (i) to glycolysis reaction conditions into R_{G} comprises contacting W with diethylene glycol in presence of a catalyst, the catalyst being selected from the group consisting of an alkali metal compounds, an alkaline earth metal compound and a mixture thereof, more preferably an alkali metal compound, more preferably alkali metal hydroxide. More preferably, the catalyst is potassium hydroxide or sodium hydroxide, more preferably potassium hydroxide.

Preferably, the hydrolysis in R_{H} according to (ii.1d.3) is performed at a temperature in the range of from 150 to 350 °C, more preferably in the range of from 210 to 290 °C, more preferably in the range of from 230 to 270 °C.

Preferably, the hydrolysis is performed in R_{H} according to (ii.1d.3) under autogenous pressure, more preferably at a pressure in the range of from 20 to 70 bar(abs), more preferably in the range of from 25 to 65 bar(abs), more preferably in the range of from 28 to 60 bar(abs).

In the context of the present invention, it is noted that any hydroalcoholysis reaction or glycolysis followed by hydrolysis known in the art can be used for performing step (ii) of the process of the present invention. For example, hydrolysis combined with glycolysis can be performed as described in US 5714523, W O2021/023889 A1, WO 2022/063764 A1 and US 5691389 A.

In the context of the present invention, preferably, the at least one polyamine obtained according to (ii) is selected from the group consisting of monomeric methylene diphenylene diamine, polymethylene polyphenylene polyamine, a mixture of monomer methylene diphenylene diamine and polymethylene polyphenylene polyamine, toluenediamine (TDA), such as TDA isomers or isomers mixture, isomers of xylylenediamine (XDA), isomers of diaminobenzene, 2,6-xylidine, naphthylene-1,5-diamine (1,5-NDA), 1,4-diaminobutane, 1,5-diaminopentane (PDA), 1,6-diaminohexane (HDA), 1,8-diaminooctane, 1,9-diaminononane, 1,10-diaminodecane, 2,2-dimethyl-1,5-diaminopentane, 2-methyl-1,5-pentanediamine (MPDA), 2,4,4(or 2,2,4)-trimethyl-1,6-diaminohexane (TMDA), 1,3- and 1,4-diaminocyclohexane, 1-amino-3,3,5-trimethyl-5-ami-nomethylcyclohexane (IPDA), 2,4- or 2,6-diamino-1-methylcyclohexane (H6-TDA), 1-amino-1-methyl-4(3)-aminomethylcyclohexane (AMCA), 1,3- bis(aminomethyl)cyclohexane, 1,4-bis(aminomethyl)cyclohexane, bis(aminomethyl)norbornane (NBDA), 4,4'-diaminodicyclohexylmethane, 2,4'-diaminodicyclohexylmethane, triaminocyclohexane, tris(aminomethyl)cyclohexane, triaminomethylcyclohexane, 1,8-diamino-4-(aminomethyl)octane, undecane-1,6,11-triamine, 1,7-diamino-4-(3-aminopropyl)heptane, 1,6-diamino-3-(aminomethyl)hexane and 1,3,5-tris(aminomethyl)cyclohexane. It is more preferred that the at least one polyamine obtained according to (ii) is selected from the group consisting of monomeric methylene diphenylene diamine, polymethylene polyphenylene polyamine, a mixture of monomeric methylene diphenylene diamine and polymethylene polyphenylene polyamine, and toluenediamine (TDA).

It is preferred that the at least one polyamine obtained according to (ii) is toluenediamine (TDA), more preferably TDA comprises a mixture of 2,4-TDA (80 wt.-%) and 2'6-TDA (20 wt.-%).

Alternatively, it is preferred that the at least one polyamine obtained according to (ii) is selected from the group consisting of monomeric methylene diphenylene diamine, polymethylene polyphenylene polyamine, and a mixture of monomeric methylene diphenylene diamine and polymethylene polyphenylene polyamine, more preferably selected from the group consisting of monomeric methylene diphenylene diamine and a mixture of monomeric methylene diphenylene diamine and polymethylene polyphenylene polyamine.

Preferably the monomeric methylene diphenylene diamine (mMDA) comprises, more preferably consists of, one or more 4,4'-diaminodiphenylmethane, 2,2'- diaminodiphenylmethane and 2,4'-diaminodiphenylmethane.

Alternatively, it is preferred that the at least one polyamine obtained according to (ii) is MDA, namely a mixture of monomeric methylene diphenylene diisocyanate (mMDA) and polymethylene polyphenylene polyisocyanate (PMDA).

In the context of the present invention, when the term "MDA" is used, it encompasses mMDA and PMDA.

### Step (iii)

Preferably, (iii) comprises
passing S1 obtained according to (ii) through U_{R}, being a solid oxide electrolysis cell (SOEC), obtaining a stream S_{CO} comprising CO.

Preferably, (iii) is performed as described in WO 2014/154253.

Preferably, (iii) comprises
(iii.1) heating S1 obtained according to (ii) in a heat exchanger;
(iii.2) passing a portion of the heated stream S1 obtained according to (iii.1) at the cathode side of the solid oxide electrolysis cell (SOEC), obtaining a stream S11 comprising CO and CO₂;
(iii.3) passing S11 through a CO₂/CO separation unit for adsorbing CO₂, obtaining a stream S_{CO}, depleted in CO₂, comprising CO;
(iii.4) compressing S_{CO}.

Preferably, the weight ratio of CO to CO₂ in stream S11 is in the range of from 1:4 to 4:1.

Preferably, the CO₂/CO separation unit for adsorbing CO₂ is one or more of an amine scrubbing unit, a membrane separation unit and a pressure-swing adsorption unit, more preferably an amine scrubbing unit for adsorbing CO₂.

Preferably, (iii) further comprises
(iii.5) passing a portion of the heated stream S1 obtained according to (iii.1) at the anode side of the solid oxide electrolysis cell (SOEC), obtaining a stream S12 comprising CO₂ and O₂;
(iii.6) passing S12 through an amine scrubbing unit for adsorbing CO₂, obtaining a stream S121, depleted in CO₂, comprising O₂;
(iii.7) compressing S121.

Preferably, from 85 to 100 weight-%, more preferably from 90 to 100 weight-%, more preferably from 95 to 100 weight-%, more preferably from 98 to 100 weight-%, more preferably from 99 to 100 weight-%, of S_{CO} obtained from SOEC consists of CO.

Preferably, the process further comprises recycling S121 for preparing chlorine.

As an alternative, (iii) preferably comprises
passing and contacting S1 obtained according to (ii) with a stream S10 comprising H₂ through U_{R}, being a reaction unit for a reverse water gas shift reaction, obtaining a stream S_{CO} comprising CO and obtaining a stream SH comprising H₂O.

Preferably, the reverse water gas shift reaction performed in U_{R} according to (iii) is performed at a temperature in the range of from 500 to 1200 °C, more preferably in the range of from 700 to 1100 °C, more preferably in the range of from 800 to 1000 °C.

Preferably, the reverse water gas shift reaction performed in U_{R} according to (iii) is performed at a pressure in the range of from 0.5 to 20 bar(abs), more preferably in the range of from 2 to 18 bar(abs), more preferably in the range of from 2 to 15 bar(abs).

The reverse water gas shift reaction are preferably performed according to known processes in the art such as for example those defined in E.Rezaei, S. Dzuryk "Techno-economic comparison of reverse water gas shift reaction to steam and dry methane reforming reactions for syngas production", Chemical Engineering Research and Design, Vol 144 (2019), S. 354-369, EP2175986, CN103183346 and US8946308.

Preferably, (iii) comprises
(iii.1') passing and contacting S1 obtained according to (ii) with a stream S10 comprising H₂ into U_{R} being a reaction unit for a reverse water gas shift reaction, obtaining a stream S13 comprising CO, CO₂, H₂ and H₂O;
(iii.2')cooling the stream S13 obtained according to (iii.1'), in a condensation unit CU, obtaining a gaseous stream S23, depleted in water, compared to S13, comprising CO and H₂, and a liquid stream SH comprising H₂O;
(iii.3') passing the stream S23 obtained according to (iii.2') into a unit for removing CO₂, more preferably an amine scrubbing unit, obtaining a stream S131, depleted in CO₂ compared to S23, comprising CO and H₂;
(iii.4') passing the stream S131 obtained according to (iii.3') into a purification unit for removing H₂, obtaining a stream S_{CO}, depleted in H₂ compared to S131, comprising CO.

Preferably, the condensation unit in (iii.2') comprises a condensation mean and one or more heat exchangers suitable for cooling down and condensation, such as plate, shell-and-tube and air cooler.

Preferably, the amine scrubbing unit in (iii.3') comprises absorption and desorption column.

Preferably, the purification unit in (iii.4') a CO-H₂ separation cold box.

Preferably, at least a portion of the CO₂, more preferably the CO₂, removed from S23 by the amine scrubbing unit is recycled as a portion of S1.

Preferably, at least a portion of the H₂, more preferably the H₂, removed from S131 by the purification unit is recycled as a portion of S10.

Preferably, the weight ratio of CO to H₂ in S13 obtained from (iii.1') is in the range of from 1:1 to 30:1, preferably in the range of from 2:1 to 20:1, more preferably in the range of from 5:1 to 15:1.

Preferably, from 85 to 100 weight-%, more preferably from 90 to 100 weight-%, more preferably from 95 to 100 weight-%, more preferably from 98 to 100 weight-%, of S13 obtained from (iii.1') consists of CO and H₂.

Preferably, from 85 to 100 weight-%, more preferably from 90 to 100 weight-%, more preferably from 95 to 100 weight-%, more preferably from 98 to 100 weight-%, of S_{CO} obtained from (iii), more preferably (iii.4'), consists of CO. More preferably, S_{CO} consists essentially of, more preferably of CO.

### Step (iv)

Preferably, (iv.1) comprises
contacting and reacting a stream comprising HCl with a stream comprising O₂ in presence of a catalyst, obtaining a stream S2 comprising Cl₂.

Preferably, the catalyst used in (iv.1) is a Ru-containing catalyst.

Preferably, the reaction in (iv.1) is performed in a reactor, more preferably a tube bundle reactor, more preferably a cooled tube bundle reactor.

Preferably, the reaction in (iv.1) is performed at a temperature in the range of from 250 to 400 °C, more preferably in the range of from 300 to 350°C.

For preparing Cl₂ according to the present invention processes well-known in the art can be used. For example, Cl₂ can be prepared according to the Deacon process as illustrated and disclosed in WO 2007/134771 A1, WO 2011/111351 A1, WO 2013/004651 A1, WO 2013/060628 A1, US 2418930, WO 2012/025483, WO 2007137685.

Alternatively, Cl₂ can be prepared via HCl electrolysis known in the art disclosed for example in US6022634, WO2003/31690, WO 1999031297, EP1103636, US3236760 and EP0004903, or via electrolysis of NaCl (chloralkali process) known in the art such as disclosed in GB 2513876 A.

As an alternative, (iv.1) preferably comprises
introducing NaCl and water in an electrolysis membrane cell, obtaining a stream S2 comprising Cl₂, a stream comprising H₂ and a stream comprising NaOH; or
introducing HCl and water in an electrolysis cell, obtaining a stream S2 comprising Cl₂ and a stream comprising H₂; or
introducing HCl and oxygen in an electrolysis cell, obtaining a stream S2 comprising Cl₂ and a stream comprising H₂O.

For obtaining S2, with HCl and O₂ in an electrolysis cell, any known process can be used, such as the process described in US 6022634, WO 2003/31690, WO 99/031297 A1.

Preferably, (iv.2) comprises
passing S_{CO} obtained according to (iii) into a reaction unit for preparing phosgene RU_{P} and contacting S_{CO} with S2 obtained according to (iv.1) in the presence of a catalyst, more preferably activated carbon, into RU_{P}, obtaining a stream S3 comprising phosgene.

Preferably, RU_{P} is a tube bundle reactor, more preferably a cooled tube bundle reactor.

Preferably, the preparation of phosgene is a heterogeneous gas phase catalysis on activated carbon.

Preferably, (iv.2) comprises
passing S_{CO} obtained according to (iii) into a reaction unit for preparing phosgene RU_{P} together with a stream S1_{CO} comprising CO, not obtained according to (iii), and contacting CO from S_{CO} and S1_{CO} with S2 obtained according to (iv.1) in the presence of a catalyst, preferably activated carbon, into RU_{P}, obtaining a stream S3 comprising phosgene.

Preferably, the stream S1_{CO} is CO.

Phosgene is preferably prepared by processes know in the art such as those disclosed in Ullmann's Encyclopedia of industrial chemistry, Chapter "Phosgene" 5th Ed., Vol. A 19, p 413 ff., VCH Verlagsgesellschaft mbH, Weinheim, 1991, and C. Ryan et.al. "Phosgene and related carbonyl halides", Elsevier Science, 1996, ISBN 978-0-08-053880-8.

Preferably, the process further comprises recycling unreacted CO from phosgenation into RU_{P} by means of an ejector.

### Step (v)

Preferably, (v) comprises
passing the at least one polyamine obtained according to (ii) as S_{PA} into a reaction unit RU and reacting S_{PA} with phosgene comprised in S3 obtained according to (iv.2), obtaining a stream SP comprising the at least one polyisocyanate and a stream S6 comprising HCl.

Preferably, the process further comprises purifying the at least one polyamine obtained according to (ii) as S_{PA} prior to passing S_{PA} into RU.

Preferably, (v) comprises
passing the at least one polyamine obtained according to (ii) as S_{PA} into a reaction unit RU together with a stream S1_{PA} comprising at least one polyamine, not obtained according to (ii.1), and reacting S_{PA} and S1_{PA} with phosgene comprised in S3 obtained according to (iv.2), obtaining a stream SP comprising the at least one polyisocyanate and a stream S6 comprising HCl.

Preferably, (v) comprises
(v.1) introducing the at least one polyamine obtained according to (ii) as S_{PA} and S3 obtained according to (iv.2), and optionally a solvent, into a mixing device comprised in the reaction unit RU, preferably a mixing nozzle, obtaining a reaction mixture;
(v.2) passing the reaction mixture obtained according to (v.1) in a reactor comprised in RU, obtaining a stream SP comprising the at least one polyisocyanate and a stream S6 comprising HCl.

Preferably, the solvent used in (v.1) is an organic solvent. More preferably, the organic solvent is toluene, monochlorobenzene or dichlorobenzene.

Preferably, in SP, the at least one polyisocyanate is a mixture of 2,4-TDI and 2,6-TDI.

Preferably, the TDI is a mixture of 2,4-TDI (80 weight-%) and 2,6-TDI (20 weight-%).

Alternatively, in SP, the at least one polyisocyanate is MDI, namely a mixture of monomeric methylene diphenylene diisocyanate (mMDI) and polymethylene polyphenylene polyisocyanate (PMDI).

Preferably the monomeric methylene diphenylene diisocyanate comprises, more preferably consists of, one or more 4,4'-methylene(diphenyl diisocyanate) (4,4'-MDI), 2,2'- methylene (diphenyl diisocyanate) (2,2'-MDI) and 2,4'- methylene (diphenyl diisocyanate) (2,4'-MDI).

Preferably, the at least one polyisocyanate comprises in SP is a monomeric methylene diphenylene diisocyanate which comprises, more preferably consists of, one or more 4,4'-methylene(diphenyl diisocyanate) (4,4'-MDI), 2,2'- methylene (diphenyl diisocyanate) (2,2'-MDI) and 2,4'- methylene (diphenyl diisocyanate) (2,4'-MDI), more preferably 4,4'-methylene(diphenyl diisocyanate) (4,4'-MDI).

In the context of the present invention, when the term "MDI" is used, it encompasses mMDI and PMDI.

Preferably, the process further comprises passing SP through one or more purification units.

Preferably, the at least one polyisocyanate comprised in SP is used for preparing at least one polyurethane.

Preferably, the process further comprises
recycling at least a portion of HCl, preferably HCl, comprised in S6 as a stream S_{HCl} used in (iv.1),
wherein (iv.1) more preferably comprises
contacting and reacting S_{HCl} comprising HCl with a stream comprising O₂ in presence of a catalyst, obtaining a stream S2 comprising Cl₂; or
introducing S_{HCl} comprising HCl and a stream comprising water in an electrolysis cell, obtaining a stream S2 comprising Cl₂ and a stream comprising H₂; or
introducing S_{HCl} comprising HCl and a stream comprising oxygen in an electrolysis cell, obtaining a stream S2 comprising Cl₂ and a stream comprising H₂O.

Preferably, the process further comprises recycling at least a portion of O₂, more preferably O₂, in S121 obtained in (iii.6) or (iii.7) as the stream comprising O₂ used in (iv.1), wherein (iv.1) comprises contacting and reacting a stream comprising HCl with a stream comprising O₂ in presence of a catalyst, obtaining a stream S2 comprising Cl₂.

In the context of the present invention, it is noted that the at least one polyol comprised in SL is an alcohol with two or more hydroxyl group. It is noted that said at least one polyol does not necessarily correspond to the polyol ("original" polyol) used for the synthesis of the polyurethane, polyurethane urea and/or polyisocyanurate comprised in W.

In the context of the present invention, a term "X is one or more of A, B and C", wherein X is a given feature and each of A, B and C stands for specific realization of said feature, is to be understood as disclosing that X is either A, or B, or C, or A and B, or A and C, or B and C, or A and B and C. In this regard, it is noted that the skilled person is capable of transfer to above abstract term to a concrete example, e.g. where X is a chemical element and A, B and C are concrete elements such as Li, Na, and K, or X is a temperature and A, B and C are concrete temperatures such as 10 °C, 20 °C, and 30 °C. In this regard, it is further noted that the skilled person is capable of extending the above term to less specific realizations of said feature, e.g. "X is one or more of A and B" disclosing that X is either A, or B, or A and B, or to more specific realizations of said feature, e.g. "X is one or more of A, B, C and D", disclosing that X is either A, or B, or C, or D, or A and B, or A and C, or A and D, or B and C, or B and D, or C and D, or A and B and C, or A and B and D, or B and C and D, or A and B and C and D.

The present invention is further illustrated by the example.

### Examples

### Example 1 Process for preparing MDI from PU waste material according to the present invention

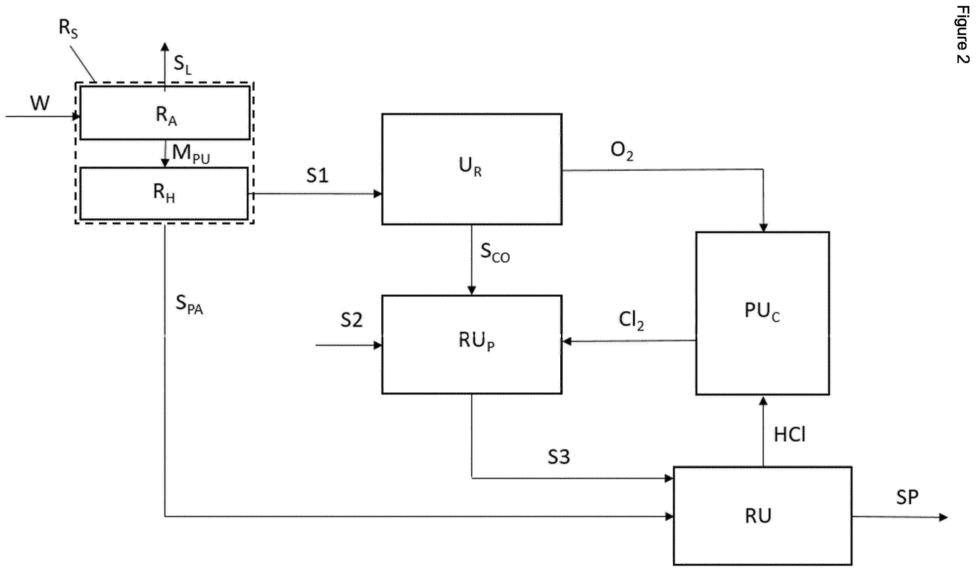

The process is based on the flow diagram of Figure 2. In a production plant, 30 kt/a (approx. 3750 kg/h) of a rigid foam waste from refrigerators (a polyurethane end-of-life foam) were processed. The rigid foam was present in the form of dried granulates and was fed into an aminolysis reactor R_{A}. There it is aminolysed with approx. 2612 kg/h n-butylamine at approx. 140°C and autogenous pressure within 1 hour residence time.

Solid components (impurities) in the reaction mixture were then filtered off. The liquid reaction mixture was mixed with approx. 31810 kg/h xylene and then the excess n-butylamine (about 1306 kg/h) was evaporated with parts of the xylene in a flash stage. The precipitation of the pol-yureas of the isocyanate component of the PU (PMDI) took place. This precipitate was filtered off and washed once with xylene (about 1000 kg/h), then dried. About 3540 kg/h of dried poly-urea was obtained.

The liquid phases from the filtration as well as from the washing (in total about 34330 kg/h) of the precipitate were distilled, obtaining a polyol phase (about 1383 kg/h), a xylene phase (about 32810 kg/h) as well as low-boiling impurities (about 134 kg/h). The xylene phase was recycled and used for dilution of the reaction mixture from the aminolysis or for washing the precipitate. Such polyol phase can be used alone or in parts in the preparation of new polyurethanes, if necessary after optional further purification and optional separation.

The dried polyurea was hydrolyzed in a hydrolysis reactor R_{H} with approx. 640 kg/h water at about 250°C under autogenous pressure (approx. 39 bar) with a residence time of about 1h. After depressurization, the resulting CO₂ (approx. 785 kg/h), the excess water (approx. 321 kg/h), the released n-butylamine (approx. 1306 kg/h) and the resulting polyamine (PMDA, approx. 1768 kg/h ) were separated by distillation. A stream S_{PA} comprising the polyamine was obtained.

The CO₂ from the hydrolysis stage was fed to a high-temperature CO₂ electrolysis (SOEC) as described in principle in WO 2014/154253. In this process, the CO₂ was first preheated and about 50 % of the CO₂ was converted to CO in a SOEC stack at 800 °C on the cathode side. The current density was about 400 mA/cm² and the specific electrical energy consumption is about 2.5 kWh/Nm³ CO. After cooling of the reaction mixture, where the energy was partly used for preheating, this reaction mixture was fed to an amine scrubbing unit (acid gas removal) with monoethanolamine, where CO₂ was chemisorbed. The absorbed CO₂ was thermally separated from the amine solution and recycled to the inlet of the electrolysis. Approximately 500 kg/h of high purified CO are leaving the amine scrubbing unit.

A stream of CO₂ was fed to the anode side. Approx. 286 kg/h of oxygen are formed at the anode. The CO₂/O₂ stream was cooled and fed to an amine scrubbing unit (acid gas removal). There, CO₂ was again chemisorbed, then thermally desorbed and again used as the stream to be converted on the anode side. The purified oxygen stream of approx. 286 kg/h was compressed.

The 500 kg/h CO obtained from SOEC were compressed and fed together with approx. 1267 kg/h chlorine to a reaction unit for preparing phosgene RU_{P}. This was carried out by means of a gas phase reaction on activated carbon as catalyst in a cooled tube bundle reactor. In this process, approx. 1768 kg/h of phosgene were recovered from the reaction mixture by condensation. Unreacted CO was recycled to RU_{P} by means of an ejector.

The amine stream S_{PA} obtained from hydrolysis (1768 kg/h) was mixed with approx. 2650 kg/h monochlorobenzene (MCB) and this mixture was fed to liquid phase phosgenation in a reaction unit RU. The 1768 kg/h phosgene stream obtained from RU_{P} was mixed with about 2650 kg/h of recycled phosgene and 2950 kg/h MCB and also fed to liquid phase phosgenation in RU. The amines were converted to the corresponding isocyanates in RU. In particular, both streams were mixed in a mixing nozzle of RU and then largely converted in a stirred tank cascade of RU. The final conversion took place in a reaction column, of RU, wherein the separation of the low temperature boiling compounds (1303 kg/h of HCl, 2650 kg/h of excess phosgene and approx. 750 kg/h of monochlorobenzene) from the high temperature boiling compounds containing the isocyanates (2232 kg/h PMDI) and 4844 kg/h of solvent took place. The isocyanate-containing phase was freed from solvent in a subsequent multistage distillation and about 2230 kg/h of a crude isocyanate mixture was obtained. Such isocyanates can be further purified and fractioned to be used for the production of polyurethanes.

The stream containing the low temperature boiling compounds was fed to an absorption unit in which phosgene was washed from the gaseous HCl with approx. 1900 kg/h of cold monochlorobenzene from the solvent separation in the distillation mentioned in the preceding paragraph. The recycled phosgene stream (2650 kg/h of phosgene) was thus provided in the sump of the absorption, as well as the solvent in the phosgene-containing stream for phosgenation (2946 kg/h).

The HCl-containing gas stream after absorption was compressed to about 13 bar(gauge) in a compressor and then freed from remaining traces of phosgene and solvent in a subsequent pressure distillation process. The HCl stream thus purified (approx. 1303 kg/h) was fed to a catalytic oxidation together with a recycled stream containing approx. 325 kg/h HCl. Here, the HCl was reacted with about 571 kg/h of an oxygen-containing stream, where 285 kg/h of O₂ comes from CO₂ electrolysis and the rest being a recycled excess of O₂. The conversion took place in a cooled tube bundle reactor on a Ru-containing catalyst at temperature of a cooling medium between 300 and 350°C. In this process, about 80% of the HCl was converted. The reaction mixture contains 1267 kg/h chlorine (Cl₂), about 325 kg/h unreacted HCl, 285 kg/h unreacted oxygen, and 321 kg/h water.

The reaction mixture was cooled in several stages and partially condensed to obtain approx. 472 kg/h of a 32% by weight aqueous HCl. The non-condensed vapors are dried in a further stage with approx. 96% sulfuric acid and then compressed to approx. 37 bar in a multi-stage compressor. Liquid chlorine (1267 kg/h) was then separated from HCl (175 kg/h) and O₂ (285 kg/h) in a distillation unit. HCl and O₂ were recycled to the reaction for preparing chlorine. The chlorine was evaporated and used in phosgene synthesis in RU_{P}. The 32 % aqueous HCl was separated by distillation in a pressure swing distillation unit into an aqueous fraction of about 321 kg/h (about 0.1 wt% HCl in water) and a gaseous HCl fraction containing about 150 kg/h HCl. The gaseous HCl stream was compressed and recycled to the reaction for preparing chlorine.

The process of Example permits to prepare a total of 2230 kg/h of isocyanate MDI and 1383 kg/h of polyol from about 3750 kg/h of PU waste material. In this example, 98.4 weight-% of the obtained polyisocyanate (MDI) are obtained from the waste material.

### Description of the figures

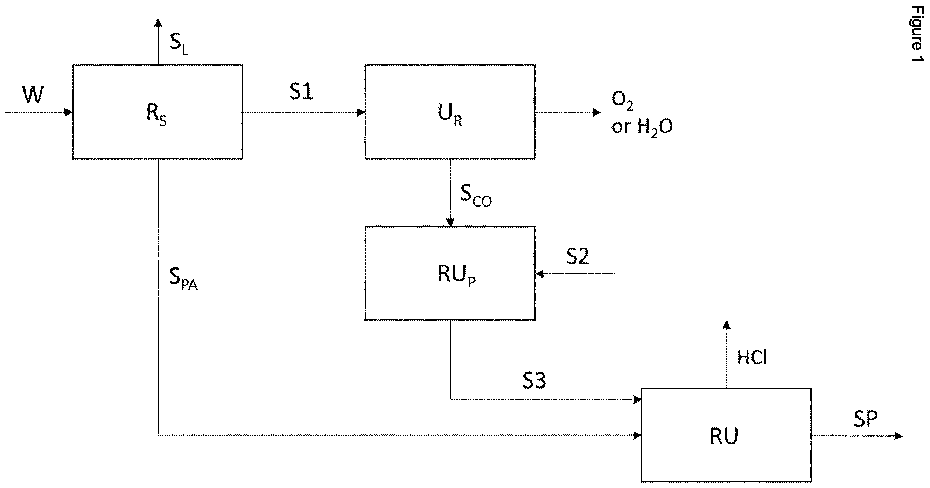

- Figure 1: is a schematic representation of a production unit used for the process according to embodiments of the invention.

The production unit comprises a solvolysis zone R_{S} for the solvolysis of a solid material W, a CO₂-reduction unit U_{R}, a reaction unit for preparing phosgene RU_{P}, and a reaction unit RU for preparing at least one polyisocyanate. The solid material W, comprising at least one polymer, selected from the group consisting of polyurethane, polyurethane urea, polyisocyanurate and a mixture thereof, is introduced and subjected to solvolysis reaction conditions into R_{S} obtaining a stream S_{PA} comprising the corresponding at least one polyamine, a stream SL comprising at least one polyol and a stream S1 comprising CO₂. S1 is passed through the CO₂-reduction unit U_{R} obtaining a stream S_{CO} comprising CO. Phosgene is also prepared; to do so the stream S_{CO} is passed into RU_{P} and contacted to S2 comprising chlorine (Cl₂), obtaining a stream S3 comprising phosgene. The stream S_{PA} is passed into RU and S_{PA} and phosgene comprised in S3 are reacted to form the stream SP comprising the corresponding at least one polyisocyanate.
- Figure 2: is a schematic representation of a production unit used for the process according to preferred embodiments of the invention.

The production unit comprises a solvolysis zone R_{S} for the solvolysis of a solid material W comprising a reaction unit R_{A} for aminolysis and a hydrolysis reactor unit R_{H}, R_{H} being located downstream of R_{A}, a CO₂-reduction unit U_{R}, a reaction unit for preparing phosgene RU_{P}, a Cl₂ production unit PU_{C} and a reaction unit RU for preparing at least one polyisocyanate. The solid material W, comprising at least one polymer, selected from the group consisting of polyurethane, polyurethane urea, polyisocyanurate and a mixture thereof, is introduced and subjected to solvolysis reaction conditions into R_{S} obtaining a stream S_{PA} comprising the corresponding at least one polyamine, a stream SL comprising at least one polyol and a stream S1 comprising CO₂. In particular, W is introduced in R_{A} and contacted with one or more amines, preferably primary amines, to obtain a mixture M_{PU} comprising at least one poly-urea containing compound and a stream SL comprising at least one polyol. The mixture M_{PU} comprising at least one poly-urea containing compound is introduced into R_{H} with water for hydrolysis, obtaining a stream S_{PA} comprising the corresponding at least one polyamine and a stream S1 comprising CO₂. S1 is passed through the CO₂-reduction unit U_{R} obtaining a stream S_{CO} comprising CO and a stream comprising O₂. Phosgene is also prepared; to do so the stream S_{CO} is passed into RU_{P} and contacted to S2 comprising chlorine (Cl₂), obtaining a stream S3 comprising phosgene. The stream S_{PA} is passed into RU and S_{PA} and phosgene comprised in S3 are reacted to form the stream SP comprising the corresponding at least one polyisocyanate. Another source of chlorine is obtained by the chlorine production unit PU_{C} to produce phosgene, wherein HCl obtained in RU is reacted with O₂ obtained from U_{R}.

### Cited literature

- WO 2021/089737 A1
- WO 2020/239716 A1
- Gregory A. Campbell et.al. "Polyurethane Foam recycling - Superheated steam hydrolysis", Environmental Science & Technology, Vol.10 (2), 1976
- US 2003/0012710 A
- US 5208379 A
- US 4317939 A
- CN 110105621 A
- DE 4217524 A1
- FR 1429011 A
- DE 4217524 A1
- EP 1149862 A1
- US 5714523
- WO2021/023889 A1
- WO 2022/063764 A1
- US 5691389 A
- WO 2014/154253
- E.Rezaei, S. Dzuryk "Techno-economic comparison of reverse water gas shift reaction to steam and dry methane reforming reactions for syngas production", Chemical Engineering Research and Design, Vol 144 (2019), S. 354-369
- EP2175986
- CN103183346
- US8946308
- WO 2007/134771 A1
- WO 2011/111351 A1
- WO 2013/004651 A1
- WO 2013/060628 A1
- US 2418930
- WO 2012/025483
- WO 2007137685
- US6022634
- WO2003/31690
- WO1999031297
- EP1103636
- US3236760
- EP0004903
- GB 2513876 A
- Ullmann's Encyclopedia of industrial chemistry, Chapter "Phosgene" 5^{th} Ed., Vol. A 19, p 413 ff., VCH Verlagsgesellschaft mbH, Weinheim, 1991, and C. Ryan et.al. "Phosgene and related carbonyl halides", Elsevier Science, 1996, ISBN 978-0-08-053880-8
- US 6022634 A
- WO 2003/31690 A2
- WO 99/031297 A1
- EP 0 976 719 A1
- WO 2012/077199 A1
- KR 2013 0120551 A.

## Claims

1. A process for preparing at least one polyisocyanate from a solid material W, the process comprising
(i) providing the solid material W comprising at least one polymer, selected from the group consisting of polyurethane, polyurethane urea, polyisocyanurate and a mixture thereof;
(ii) preparing at least one polyamine from W, comprising:
passing and subjecting W provided according to (i) to solvolysis reaction conditions into a solvolysis zone R_{S} comprising one or more reactor units, obtaining a stream S_{PA} comprising the corresponding at least one polyamine, a stream SL comprising at least one polyol and a stream S1 comprising CO₂;
(iii) passing S1 obtained according to (ii) through a CO₂-reduction unit U_{R}, obtaining a stream S_{CO} comprising CO;
(iv) preparing phosgene, comprising
(iv.1) providing a stream S2 comprising Cl₂;
(iv.2) passing S_{CO} obtained according to (iii) into a reaction unit for preparing phosgene RU_{P} and contacting S_{CO} with S2 obtained according to (iv.1) into RU_{P}, obtaining a stream S3 comprising phosgene;
(v) passing the at least one polyamine obtained according to (ii) as S_{PA} into a reaction unit RU and reacting S_{PA} with phosgene comprised in S3 obtained according to (iv.2), obtaining a stream SP comprising the corresponding at least one polyisocyanate;
wherein, in SP, the at least one polyisocyanate is TDI or MDI.

2. The process of claim 1, wherein (ii) comprises
(ii.1) passing and subjecting W provided according to (i) to hydrolysis reaction conditions into R_{S} being a hydrolysis reaction unit, obtaining a stream S_{PA} comprising the corresponding at least one polyamine, a stream SL comprising at least one polyol and a stream S1 comprising CO₂.

3. The process of claim 1, wherein (ii) comprises
(ii.1a)passing and subjecting W provided according to (i) to hydroaminolysis reaction conditions into R_{S} being a hydroaminolysis reaction unit, obtaining a stream S_{PA} comprising the corresponding at least one polyamine, a stream SL comprising at least one polyol and a stream S1 comprising CO₂.

4. The process of claim 1, wherein (ii) comprises
(ii.1b.1) passing W provided in (i) and one or more amines in a reactor unit R_{A} comprised in R_{S} and subjecting W and the one or more amines to aminolysis reaction conditions in R_{A}, obtaining a mixture M comprising at least one polyurea-containing compound, and further comprising at least one polyol;
(ii.1b.2) isolating the at least one polyurea-containing compound of M from the at least one polyol obtained according to (ii.1b.1), obtaining M_{PU} comprising the at least one polyurea-containing compound;
(ii.1b.3) passing water as stream S0 and M_{PU} obtained according to (ii.1b.2) through an hydrolysis reactor unit R_{H} comprised in R_{S} and located downstream of R_{A}, obtaining a stream S_{PA} comprising the corresponding at least one polyamine and a stream S1 comprising CO₂.

5. The process of claim 4, wherein, in (ii.1b.1), the ratio of the weight of the solid material W introduced into R_{A} relative to the weight of the one or more amines introduced into R_{A} is in the range of from 1:100 to 1:1, preferably in the range of from 1:40 to 1:3, more preferably in the range of from 1:27 to 1:5.

6. The process of claim 1, wherein (ii) comprises
(ii.1c)passing and subjecting W provided according to (i) to hydroalcoholysis reaction conditions into R_{S} being a hydroalcoholysis reaction unit, obtaining a stream S_{PA} comprising the corresponding at least one polyamine, a stream SL comprising at least one polyol and a stream S1 comprising CO₂.

7. The process of claim 1, wherein (ii) comprises
(ii.1d.1) passing W provided in (i) and one or more alcohols, preferably diethylene glycol, in a reactor unit R_{G} comprised in R_{S} and subjecting W and the one or more alcohols to alcoholysis reaction conditions, preferably glycolysis reaction conditions, in R_{A}, obtaining a mixture M comprising at least one carbamate-containing compound, and further comprising at least one polyol;
(ii.1d.2) isolating the at least one carbamate-containing compound of M from the at least one polyol obtained according to (ii.1d.1), obtaining M_{PU} comprising the at least one carbamate-containing compound and a stream SL comprising the at least one polyol;
(ii.1d.3) passing water as stream S0 and M_{PU} obtained according to (ii.1d.2) through an hydrolysis reactor unit R_{H} comprised in R_{S} and located downstream of R_{A}, obtaining a stream S_{PA} comprising the corresponding at least one polyamine and a stream S1 comprising CO₂.

8. The process of any one of claims 1 to 7, wherein (iii) comprises
passing S1 obtained according to (ii) through U_{R}, being a solid oxide electrolysis cell (SOEC), obtaining a stream S_{CO} comprising CO.

9. The process of any one of claims 1 to 7, wherein (iii) comprises
passing and contacting S1 obtained according to (ii) with a stream S10 comprising H₂ through U_{R}, being a reaction unit for a reverse water gas shift reaction, obtaining a stream S_{CO} comprising CO and obtaining a stream SH comprising H₂O.

10. The process of any one of claims 1 to 9, wherein (iv.1) comprises
contacting and reacting a stream comprising HCl with a stream comprising O₂ in presence of a catalyst, obtaining a stream S2 comprising Cl₂; or
introducing NaCl and water in an electrolysis membrane cell, obtaining a stream S2 comprising Cl₂, a stream comprising H₂ and a stream comprising NaOH; or
introducing HCl and water in an electrolysis cell, obtaining a stream S2 comprising Cl₂ and a stream comprising H₂; or
introducing HCl and oxygen in an electrolysis cell, obtaining a stream S2 comprising Cl₂ and a stream comprising H₂O.

11. The process of any one of claims 1 to 10, wherein (v) comprises
passing the at least one polyamine obtained according to (ii) as S_{PA} into a reaction unit RU and reacting S_{PA} with phosgene comprised in S3 obtained according to (iv.2), obtaining a stream SP comprising the at least one polyisocyanate and a stream S6 comprising HCl.

12. The process of claim 11, wherein (v) comprises
(v.1) introducing the at least one polyamine obtained according to (ii) as S_{PA} and S3 obtained according to (iv.2), and optionally a solvent, into a mixing device comprised in the reaction unit RU, preferably a mixing nozzle, obtaining a reaction mixture;
(v.2) passing the reaction mixture obtained according to (v.1) in a reactor comprised in RU, obtaining a stream SP comprising the at least one polyisocyanate and a stream S6 comprising HCl.

13. The process of claim 11 or 12, further comprising
recycling at least a portion of HCl, preferably HCl, comprised in S6 as a stream S_{HCl} used in (iv.1).

## Patentansprüche

1. Verfahren zur Herstellung von mindestens einem Polyisocyanat aus einem festen Material W, wobei das Verfahren umfasst:
(i) Bereitstellen des festen Materials W, das mindestens ein Polymer umfasst, ausgewählt aus der Gruppe bestehend aus Polyurethan, Polyurethan-Harnstoff, Polyisocyanurat und einer Mischung daraus;
(ii) Herstellen von mindestens einem Polyamin aus W, umfassend: Leiten und Unterwerfen von W, bereitgestellt gemäß (i), unter Solvolyse-Reaktionsbedingungen in eine Solvolysezone R_{S}, die eine oder mehrere Reaktoreinheiten umfasst, wobei ein Strom S_{PA} erhalten wird, der das entsprechende mindestens eine Polyamin umfasst, ein Strom SL, der mindestens ein Polyol umfasst, und ein Strom S1, der CO₂ umfasst;
(iii) Leiten von S1, erhalten gemäß (ii), durch eine CO₂-Reduktionseinheit U_{R}, wobei ein Strom S_{CO} erhalten wird, der CO umfasst;
(iv) Herstellen von Phosgen, umfassend:
(iv.1) Bereitstellen eines Stroms S2, der Cl₂ umfasst;
(iv.2) Leiten von S_{CO}, erhalten gemäß (iii), in eine Reaktionseinheit zur Phosgenherstellung RU_{P} und Inkontaktbringen von S_{CO} mit S2, erhalten gemäß (iv.1), in RU_{P}, wobei ein Strom S3 erhalten wird, der Phosgen umfasst;
(v) Leiten des mindestens einen Polyamins, erhalten gemäß (ii), als S_{PA} in eine Reaktionseinheit RU und Umsetzen von S_{PA} mit Phosgen, enthalten in S3, erhalten gemäß (iv.2), wobei ein Strom SP erhalten wird, der das entsprechende mindestens eine Polyisocyanat umfasst;
wobei in SP das mindestens eine Polyisocyanat TDI oder MDI ist.

2. Verfahren nach Anspruch 1, wobei (ii) umfasst:
(ii.1) Leiten und Unterwerfen von W, bereitgestellt gemäß (i), unter Hydrolysereaktionsbedingungen in R_{S}, wobei R_{S} eine Hydrolysereaktionseinheit ist, wobei ein Strom S_{PA} erhalten wird, der das entsprechende mindestens eine Polyamin umfasst, ein Strom SL, der mindestens ein Polyol umfasst, und ein Strom S1, der CO₂ umfasst.

3. Verfahren nach Anspruch 1, wobei (ii) umfasst:
(ii.1a) Leiten und Unterwerfen von W, bereitgestellt gemäß (i), unter Hydroaminolyse-Reaktionsbedingungen in R_{S}, wobei R_{S} eine Hydroaminolyse-Reaktionseinheit ist, wobei ein Strom S_{PA} erhalten wird, der das entsprechende mindestens eine Polyamin umfasst, ein Strom SL, der mindestens ein Polyol umfasst, und ein Strom S1, der CO₂ umfasst.

4. Verfahren nach Anspruch 1, wobei (ii) umfasst:
(ii.1b.1) Leiten von W, bereitgestellt gemäß (i), und einem oder mehreren Aminen in eine Reaktionseinheit R_{A}, die in R_{S} enthalten ist, und Unterwerfen von W und dem einen oder mehreren Aminen unter Aminolyse-Reaktionsbedingungen in R_{A}, wobei eine Mischung M erhalten wird, die mindestens eine Polyharnstoff-haltige Verbindung und weiterhin mindestens ein Polyol umfasst;
(ii.1b.2) Isolieren der mindestens einen Polyharnstoff-haltigen Verbindung der Mischung M von dem mindestens einen Polyol, erhalten gemäß (ii.1b.1), wobei M_{PU} erhalten wird, die die mindestens eine Polyharnstoff-haltige Verbindung umfasst;
(ii.1b.3) Leiten von Wasser als Strom S0 und von M_{PU}, erhalten gemäß (ii.1b.2), durch eine Hydrolysereaktoreinheit R_{H}, die in R_{S} enthalten und stromabwärts von R_{A} angeordnet ist, wobei ein Strom S_{PA} erhalten wird, der das entsprechende mindestens eine Polyamin umfasst, und ein Strom S1, der CO₂ umfasst.

5. Verfahren nach Anspruch 4, wobei in (ii.1b.1) das Verhältnis des Gewichts des festen Materials W, das in R_{A} eingeführt wird, relativ zu dem Gewicht des einen oder mehrerer Amine, die in R_{A} eingeführt werden, im Bereich von 1:100 bis 1:1 liegt, vorzugsweise im Bereich von 1:40 bis 1:3, bevorzugter im Bereich von 1:27 bis 1:5.

6. Verfahren nach Anspruch 1, wobei (ii) umfasst:
(ii.1c) Leiten und Unterwerfen von W, bereitgestellt gemäß (i), unter Hydroalkoholyse-Reaktionsbedingungen in R_{S}, wobei R_{S} eine Hydroalkoholyse-Reaktionseinheit ist, wobei ein Strom S_{PA} erhalten wird, der das entsprechende mindestens eine Polyamin umfasst, ein Strom SL, der mindestens ein Polyol umfasst, und ein Strom S1, der CO₂ umfasst.

7. Verfahren nach Anspruch 1, wobei (ii) umfasst:
(ii.1d.1) Leiten von W, bereitgestellt gemäß (i), und einem oder mehreren Alkoholen, vorzugsweise Diethylenglykol, in eine Reaktionseinheit R_{G}, die in R_{S} enthalten ist, und Unterwerfen von W und dem einen oder mehreren Alkoholen unter Alkoholyse-Reaktionsbedingungen, vorzugsweise Glykolyse-Reaktionsbedingungen, in R_{A}, wobei eine Mischung M erhalten wird, die mindestens eine Carbamat-haltige Verbindung und weiterhin mindestens ein Polyol umfasst;
(ii.1d.2) Isolieren der mindestens einen Carbamat-haltigen Verbindung der Mischung M von dem mindestens einen Polyol, erhalten gemäß (ii.1d.1), wobei M_{PU} erhalten wird, die die mindestens eine Carbamat-haltige Verbindung umfasst, und ein Strom SL, der das mindestens eine Polyol umfasst;
(ii.1d.3) Leiten von Wasser als Strom S0 und von M_{PU}, erhalten gemäß (ii.1d.2), durch eine Hydrolysereaktoreinheit R_{H}, die in R_{S} enthalten und stromabwärts von R_{A} angeordnet ist, wobei ein Strom S_{PA} erhalten wird, der das entsprechende mindestens eine Polyamin umfasst, und ein Strom S1, der CO₂ umfasst.

8. Verfahren nach einem der Ansprüche 1 bis 7, wobei (iii) umfasst:
Leiten von S1, erhalten gemäß (ii), durch U_{R}, wobei U_{R} eine Festoxid-Elektrolysezelle (SOEC) ist, wobei ein Strom S_{CO} erhalten wird, der CO umfasst.

9. Verfahren nach einem der Ansprüche 1 bis 7, wobei (iii) umfasst:
Leiten und Inkontaktbringen von S1, erhalten gemäß (ii), mit einem Strom S10, der H₂ umfasst, durch U_{R}, wobei U_{R} eine Reaktionseinheit für eine umgekehrte Wassergas-Shift-Reaktion ist, wobei ein Strom S_{CO} erhalten wird, der CO umfasst, und ein Strom SH, der H₂O umfasst.

10. Verfahren nach einem der Ansprüche 1 bis 9, wobei (iv.1) umfasst:
Inkontaktbringen und Umsetzen eines Stroms, der HCl umfasst, mit einem Strom, der O₂ umfasst, in Gegenwart eines Katalysators, wobei ein Strom S2 erhalten wird, der Cl₂ umfasst; oder
Einleiten von NaCl und Wasser in eine Elektrolyse-Membranzelle, wobei ein Strom S2 erhalten wird, der Cl₂ umfasst, ein Strom, der H₂ umfasst, und ein Strom, der NaOH umfasst; oder
Einleiten von HCl und Wasser in eine Elektrolysezelle, wobei ein Strom S2 erhalten wird, der Cl₂ umfasst, und ein Strom, der H₂ umfasst; oder
Einleiten von HCl und Sauerstoff in eine Elektrolysezelle, wobei ein Strom S2 erhalten wird, der Cl₂ umfasst, und ein Strom, der H₂O umfasst.

11. Verfahren nach einem der Ansprüche 1 bis 10, wobei (v) umfasst:
Leiten des mindestens einen Polyamins, erhalten gemäß (ii), als S_{PA} in eine Reaktionseinheit RU und Umsetzen von S_{PA} mit Phosgen, enthalten in S3, erhalten gemäß (iv.2), wobei ein Strom SP erhalten wird, der das mindestens eine Polyisocyanat umfasst, und ein Strom S6, der HCl umfasst.

12. Verfahren nach Anspruch 11, wobei (v) umfasst:
(v.1) Einführen des mindestens einen Polyamins, erhalten gemäß (ii), als S_{PA} und von S3, erhalten gemäß (iv.2), und gegebenenfalls eines Lösungsmittels in eine Mischeinrichtung, die in der Reaktionseinheit RU enthalten ist, vorzugsweise eine Mischdüse, wobei ein Reaktionsgemisch erhalten wird;
(v.2) Leiten des Reaktionsgemischs, erhalten gemäß (v.1), in einem Reaktor, der in RU enthalten ist, wobei ein Strom SP erhalten wird, der das mindestens eine Polyisocyanat umfasst, und ein Strom S6, der HCl umfasst.

13. Verfahren nach Anspruch 11 oder 12, weiterhin umfassend:
Rückführen von mindestens einem Teil des in S6 enthaltenen HCl, vorzugsweise HCl, als Strom S_{HCl}, der in (iv.1) verwendet wird.

## Revendications

1. Procédé de préparation d'au moins un polyisocyanate à partir d'un matériau solide W, le procédé comprenant :
(i) fourniture du matériau solide W comprenant au moins un polymère choisi dans le groupe constitué de polyuréthane, polyuréthane-urée, polyisocyanurate et mélange de ceux-ci ;
(ii) préparation d'au moins une polyamine à partir de W, comprenant :
faire passer et soumettre W fourni selon (i) à des conditions de réaction de solvolyse dans une zone de solvolyse R_{S} comprenant une ou plusieurs unités de réacteur, obtenant un flux S_{PA} comprenant la ou les polyamines correspondantes, un flux SL comprenant au moins un polyol et un flux S1 comprenant du CO₂ ;
(iii) passage de S1 obtenu selon (ii) à travers une unité de réduction de CO₂ U_{R}, obtenant un flux S_{CO} comprenant du CO ;
(iv) préparation de phosgène, comprenant :
(iv.1) fourniture d'un flux S2 comprenant Cl₂ ;
(iv.2) passage de S_{CO} obtenu selon (iii) dans une unité de réaction pour la préparation du phosgène RU_{P} et mise en contact de S_{CO} avec S2 obtenu selon (iv.1) dans RU_{P}, obtenant un flux S3 comprenant du phosgène ;
(v) passage d'au moins une polyamine obtenue selon (ii) en tant que S_{PA} dans une unité de réaction RU et réaction de S_{PA} avec le phosgène contenu dans S3 obtenu selon (iv.2), obtenant un flux SP comprenant le ou les polyisocyanates correspondants ;
dans lequel, dans SP, le ou les polyisocyanates sont le TDI ou le MDI.

2. Procédé selon la revendication 1, dans lequel (ii) comprend :
(ii.1) faire passer et soumettre W fourni selon (i) à des conditions de réaction d'hydrolyse dans R_{S}, R_{S} étant une unité de réaction d'hydrolyse, obtenant un flux S_{PA} comprenant la ou les polyamines correspondantes, un flux SL comprenant au moins un polyol et un flux S1 comprenant du CO₂.

3. Procédé selon la revendication 1, dans lequel (ii) comprend :
(ii.1a) faire passer et soumettre W fourni selon (i) à des conditions de réaction d'hydroaminolyse dans R_{S}, R_{S} étant une unité de réaction d'hydroaminolyse, obtenant un flux S_{PA} comprenant la ou les polyamines correspondantes, un flux SL comprenant au moins un polyol et un flux S1 comprenant du CO₂.

4. Procédé selon la revendication 1, dans lequel (ii) comprend :
(ii.1b.1) faire passer W fourni selon (i) et une ou plusieurs amines dans une unité de réacteur R_{A} comprise dans R_{S} et soumettre W et la ou les amines à des conditions de réaction d'aminolyse dans R_{A}, obtenant un mélange M comprenant au moins un composé contenant du polyurée et comprenant en outre au moins un polyol ;
(ii.1b.2) isoler le ou les composés contenant du polyurée de M du ou des polyols obtenus selon (ii.1b.1), obtenant M_{PU} comprenant le ou les composés contenant du polyurée ;
(ii.1b.3) faire passer de l'eau en tant que flux S0 et M_{PU} obtenu selon (ii.1b.2) à travers une unité de réacteur d'hydrolyse R_{H} comprise dans R_{S} et disposée en aval de R_{A}, obtenant un flux S_{PA} comprenant la ou les polyamines correspondantes et un flux S1 comprenant du CO₂.

5. Procédé selon la revendication 4, dans lequel, dans (ii.1b.1), le rapport du poids du matériau solide W introduit dans R_{A} par rapport au poids de la ou des amines introduites dans R_{A} est compris dans la plage de 1:100 à 1:1, de préférence dans la plage de 1:40 à 1:3, plus préférentiellement dans la plage de 1:27 à 1:5.

6. Procédé selon la revendication 1, dans lequel (ii) comprend :
(ii.1c) faire passer et soumettre W fourni selon (i) à des conditions de réaction d'hydroalcoolyse dans R_{S}, R_{S} étant une unité de réaction d'hydroalcoolyse, obtenant un flux S_{PA} comprenant la ou les polyamines correspondantes, un flux SL comprenant au moins un polyol et un flux S1 comprenant du CO₂.

7. Procédé selon la revendication 1, dans lequel (ii) comprend :
(ii.1d.1) faire passer W fourni selon (i) et un ou plusieurs alcools, de préférence du diéthylène glycol, dans une unité de réacteur R_{G} comprise dans R_{S} et soumettre W et le ou les alcools à des conditions de réaction d'alcoolyse, de préférence des conditions de réaction de glycolyse, dans R_{A}, obtenant un mélange M comprenant au moins un composé contenant un carbamate et comprenant en outre au moins un polyol ;
(ii.1d.2) isoler le ou les composés contenant un carbamate de M du ou des polyols obtenus selon (ii.1d.1), obtenant M_{PU} comprenant le ou les composés contenant un carbamate et un flux SL comprenant le ou les polyols ;
(ii.1d.3) faire passer de l'eau en tant que flux S0 et M_{PU} obtenu selon (ii.1d.2) à travers une unité de réacteur d'hydrolyse R_{H} comprise dans R_{S} et disposée en aval de R_{A}, obtenant un flux S_{PA} comprenant la ou les polyamines correspondantes et un flux S1 comprenant du CO₂.

8. Procédé selon l'une quelconque des revendications 1 à 7, dans lequel (iii) comprend : faire passer S1 obtenu selon (ii) à travers U_{R}, U_{R} étant une cellule d'électrolyse à oxyde solide (SOEC), obtenant un flux S_{CO} comprenant du CO.

9. Procédé selon l'une quelconque des revendications 1 à 7, dans lequel (iii) comprend : faire passer et mettre en contact S1 obtenu selon (ii) avec un flux S10 comprenant H₂ à travers U_{R}, U_{R} étant une unité de réaction pour une réaction de décalage eau-gaz inverse, obtenant un flux S_{CO} comprenant du CO et un flux SH comprenant H₂O.

10. Procédé selon l'une quelconque des revendications 1 à 9, dans lequel (iv.1) comprend :
mettre en contact et faire réagir un flux comprenant HCl avec un flux comprenant O₂ en présence d'un catalyseur, obtenant un flux S2 comprenant Cl₂ ; ou
introduire NaCl et de l'eau dans une cellule d'électrolyse à membrane, obtenant un flux S2 comprenant Cl₂, un flux comprenant H₂ et un flux comprenant NaOH ; ou
introduire HCl et de l'eau dans une cellule d'électrolyse, obtenant un flux S2 comprenant Cl₂ et un flux comprenant H₂ ; ou
introduire HCl et de l'oxygène dans une cellule d'électrolyse, obtenant un flux S2 comprenant Cl₂ et un flux comprenant H₂O.

11. Procédé selon l'une quelconque des revendications 1 à 10, dans lequel (v) comprend : faire passer au moins une polyamine obtenue selon (ii) en tant que S_{PA} dans une unité de réaction RU et faire réagir S_{PA} avec le phosgène contenu dans S3 obtenu selon (iv.2), obtenant un flux SP comprenant le ou les polyisocyanates et un flux S6 comprenant HCl.

12. Procédé selon la revendication 11, dans lequel (v) comprend :
(v.1) introduire la ou les polyamines obtenues selon (ii) en tant que S_{PA} et S3 obtenu selon (iv.2), et éventuellement un solvant, dans un dispositif de mélange compris dans l'unité de réaction RU, de préférence une buse de mélange, obtenant un mélange réactionnel ;
(v.2) faire passer le mélange réactionnel obtenu selon (v.1) dans un réacteur compris dans RU, obtenant un flux SP comprenant le ou les polyisocyanates et un flux S6 comprenant HCl.

13. Procédé selon la revendication 11 ou 12, comprenant en outre :
recyclage d'au moins une partie du HCl, de préférence HCl, contenu dans S6 en tant que flux S_{HCl} utilisé dans (iv.1).
